(19) **Europäisches Patentamt European Patent Office Office européen des brevets**

(11) **EP 4 461 754 B1**

(12) **EUROPEAN PATENT SPECIFICATION**

(45) Date of publication and mention
of the grant of the patent:
**22.07.2026  Bulletin 2026/30**

(21) Application number: **23814347.3**

(22) Date of filing: **22.05.2023**

(51) International Patent Classification (IPC):
*G16C 20/10* (2019.01)     *G16C 20/30* (2019.01)
*G06N 3/02* (2006.01)     *G06F 18/2135* (2023.01)
*G06F 18/23* (2023.01)     *G16C 20/70* (2019.01)
*G16C 60/00* (2019.01)

(52) Cooperative Patent Classification (CPC):
**G06N 3/048; C08F 20/00; G06F 18/2135;
G06F 18/23; G06N 3/044; G06N 3/08; G06N 3/084;
G16C 20/10; G16C 20/30;** G16C 20/70;
G16C 60/00

(86) International application number:
**PCT/JP2023/019014**

(87) International publication number:
**WO 2024/202078 (03.10.2024 Gazette 2024/40)**

(54) **METHOD FOR MAKING PREDICTION RELATED TO ADDITION POLYMERIZATION REACTION, INFORMATION PROCESSING DEVICE, AND PROGRAM**

VERFAHREN ZUR DURCHFÜHRUNG EINER VORHERSAGE IM ZUSAMMENHANG MIT EINER ADDITIONSPOLYMERISATIONSREAKTION, INFORMATIONSVERARBEITUNGSVORRICHTUNG UND PROGRAMM

PROCÉDÉ DE RÉALISATION D'UNE PRÉDICTION LIÉE À UNE RÉACTION DE POLYMÉRISATION PAR ADDITION, DISPOSITIF DE TRAITEMENT D'INFORMATIONS ET PROGRAMME

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **24.03.2023   JP 2023048922**

(43) Date of publication of application:
**13.11.2024   Bulletin 2024/46**

(73) Proprietor: **DIC Corporation**
**Tokyo 174-8520 (JP)**

(72) Inventors:
  • **NAGAO Atsushi**
    **Takaishi-shi**
    **Osaka**
    **5920001 (JP)**
  • **MATSUFUJI Yoshimasa**
    **Takaishi-shi**
    **Osaka**
    **5920001 (JP)**
  • **SHIBAMOTO Naoki**
    **Takaishi-shi**
    **Osaka**
    **5920001 (JP)**
  • **KARIURA Shinsuke**
    **Tokyo**
    **1038233 (JP)**
  • **MATSUI Kouichi**
    **Ichihara-shi**
    **Chiba**
    **2908585 (JP)**

(74) Representative: **TBK**
    **Bavariaring 4-6**
    **80336 München (DE)**

(56) References cited:
    **WO-A1-2022/004880     JP-A- H0 628 009**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ABEYKOON CHAMIL ED - IEEE: "Design and Applications of Soft Sensors in Polymer Processing: A Review", IEEE SENSORS JOURNAL, IEEE, USA, vol. 19, no. 8, 15 April 2019 (2019-04-15), pages 2801 - 2813, XP011715326, ISSN: 1530-437X, [retrieved on 20190315], DOI: 10.1109/JSEN.2018.2885609
- YIN ZHANGQI ET AL: "Forecasting the Intrinsic Viscosity of Polyester Based on Improved Extreme Learning Machine", 2019 IEEE INTERNATIONAL CONFERENCE ON ARTIFICIAL INTELLIGENCE AND COMPUTER APPLICATIONS (ICAICA), IEEE, 29 March 2019 (2019-03-29), pages 241 - 246, XP033633381, DOI: 10.1109/ICAICA.2019.8873494
- JINTAO SHI ET AL: "Online prediction based on the Copula function for the intrinsic viscosity of polyester fibre", CANADIAN JOURNAL OF CHEMICAL ENGINEERING, WILEY SUBSCRIPTION SERVICES, INC., A WILEY COMPANY, HOBOKEN, USA, vol. 101, no. 3, 30 May 2022 (2022-05-30), pages 1440 - 1454, XP072541996, ISSN: 0008-4034, DOI: 10.1002/CJCE.24431
- GENG JUNXIAN ET AL: "Fractal-based combined kernel function model for the polyester polymerization process", 2021 33RD CHINESE CONTROL AND DECISION CONFERENCE (CCDC), IEEE, 22 May 2021 (2021-05-22), pages 656 - 661, XP034032008, DOI: 10.1109/CCDC52312.2021.9601586
- "Materials Informatics: A Super Introduction to Machine Learning for Materials Development, First Edition", 1 January 2019, NIKKAN KOGYO SHIMBUN, JP, ISBN: 978-4-526-07986-3, article YUUMA IWASAKI: "2.20 Data Preprocessing", pages: 52 - 54, XP009559444

**Description**

Technical Field

The present disclosure relates to a method for performing prediction related to an addition polymerization reaction, an information processing device, and a program. The present application claims priority based on Japanese Patent Application No. 2023-048922 filed in Japan on March 24, 2023.

Background Art

Conventionally, methods for performing prediction related to chemical reactions have been developed (for example, PTL 1).

Citation List

Patent Literature

PTL 1: WO 2003/026791
WO 2022/004880 A1 discloses a prediction device for predicting a performance value indicating performance of a polymer in a polymerization tank after raw materials are added therein when manufacturing the polymer. The prediction device is provided with: an acquisition unit that acquires, as a prediction observation value, an observation value observed as a value related to manufacturing of a polymer in current manufacturing of the polymer; and a prediction unit that, by using a relation between an observation value obtained in past manufacturing of a polymer of the same kind as the polymer and a performance value of the polymer at a predetermined timing in the past manufacturing, predicts the performance value of the currently manufactured polymer at the predetermined timing from the prediction observation value acquired by the acquisition unit.

ABEYKOON CHAMIL ED - IEEE: "Design and Applications of Soft Sensors in Polymer Processing: A Review", IEEE SENSORS JOURNAL, IEEE, USA, vol. 19, no. 8, 15 April 2019 (2019-04-15), pages 2801-2813, ISSN: 1530-437X, DOI:10.1109/JSEN.2018.2885609 discloses a comprehensive review on the use of soft sensing techniques in polymer processing applications while identifying their capabilities and limitations, and discusses the importance of developing of such soft sensing techniques (together with some sort of built-on intelligence) for the advancement of process monitoring, while indicating some of the possible directions for future industry.

YIN ZHANGQI ET AL: "Forecasting the Intrinsic Viscosity of Polyester Based on Improved Extreme Learning Machine", 2019 IEEE INTERNATIONAL CONFERENCE ON ARTIFICIAL INTELLIGENCE AND COMPUTER APPLICATIONS (ICAICA), IEEE, 29 March 2019 (2019-03-29), pages 241-246, DOI: 10.1109/ICAICA.2019.8873494, discloses a forecasting model of intrinsic viscosity of polyester based on an extreme learning machine.

JINTAO SHI ET AL: "Online prediction based on the Copula function for the intrinsic viscosity of polyester fibre", CANADIAN JOURNAL OF CHEMICAL ENGINEERING, WILEY SUBSCRIPTION SERVICES, INC., A WILEY COMPANY, HOBOKEN, USA, vol. 101, no. 3, 30 May 2022 (2022-05-30), pages 1440-1454, ISSN: 0008-4034, DOI: 10.1002/CJCE.24431, discloses introducing the Copula function into the modelling of the polymerization process.

GENG JUNXIAN ET AL: "Fractal-based combined kernel function model for the polyester polymerization process", 2021 33RD CHINESE CONTROL AND DECISION CONFERENCE (CCDC), IEEE, 22 May 2021 (2021-0522), pages 656-661, DOI:10.1109/CCD052312.2021.9601586 introduces the fractal dimension feature selection method and combines the kernel function model based on STL decomposition (Seasonal-Trend decomposition procedure based on Loess) for the polyester polymerization process.

Summary of Invention

Technical Problem

The technique described in PTL 1 has described the use of modeling techniques such as neural networks, partial least squares, and principal component regression to optimize the control of reactor systems. However, specific design methods and optimization in performing predictions related to addition polymerization reactions have not been considered, and there has been room for improvement in the prediction technology related to the addition polymerization reactions.

An object of the present disclosure made in view of such circumstances is to improve the prediction technology related to the addition polymerization reactions.

Solution to Problem

According to the present invention, a method for performing prediction related to an addition polymerization reaction, an information processing device, and a non-transitory computer-readable recording medium are provided as specified by the appended claims. The invention is set out in the independent claims. The dependent claims define advantageous embodiments. The embodiments that do not fall under the scope of the claims are to be interpreted as examples useful for understanding the disclosure.

Advantageous Effects of Invention

According to the method for performing prediction related to an addition polymerization reaction, the information processing device, and the program of the present invention, the prediction technology related to the addition polymerization reaction can be improved.

Brief Description of Drawings

FIG. 1 is a block diagram illustrating the schematic configuration of an information processing device according to the present embodiment.
FIG. 2 is a flowchart illustrating operations of the information processing device according to the present embodiment.
FIG. 3 is concept of an addition polymerization reaction process according to the present embodiment.
FIG. 4A is a view illustrating the time transition of each piece of data pertaining to a temperature rising process and a dropwise addition process.
FIG. 4B illustrates the time transition of categories related to the temperature rising process and the dropwise addition process.
FIG. 5 illustrates one example of the accuracy verification result of a prediction model according to the present embodiment.
FIG. 6 illustrates one example of the accuracy verification result of the prediction model according to the present embodiment.
FIG. 7 is one example of the conceptual view of the prediction model in the present embodiment.

Description of Embodiments

Hereinafter, a method for performing prediction related to an addition polymerization reaction, an information processing device, and a program in an embodiment of the present disclosure will be described with reference to the drawings. A prediction target according to the embodiment of the present disclosure includes both batch reaction and continuous reaction. Examples of the main polymer materials synthesized by the addition polymerization reaction according to the present embodiment include acrylic, poly(meth)acrylic acid esters, polyethylene, polypropylene, polystyrene, polyvinyl chloride, polyvinyl acetate, polyvinylidene chloride, polyacrylonitrile, and polytetrafluoroethylene. For example, the addition polymerization reaction according to the present embodiment includes an addition polymerization reaction of the acrylic.

In each of the drawings, identical or equivalent parts are assigned the same symbols. In the description of the present embodiment, descriptions of the identical or equivalent parts are omitted or simplified as appropriate.

First, the overview of the present embodiment will be described. The method for performing prediction related to an addition polymerization reaction in the present embodiment is executed by an information processing device 10. The information processing device 10 trains a prediction model based on actual data including a plurality of explanatory factors and an objective factor related to the addition polymerization reaction. The information processing device 10 predicts the objective factor during the addition polymerization reaction based on the explanatory factors related to the addition polymerization reaction by the trained prediction model. The explanatory factors include a plurality of feature values obtained by clustering analysis of time-series data from a plurality of measurement instruments at a temperature rising process and a dropwise addition process. The objective factor is characterized by including at least either an NV or a solution viscosity. The NV stands for Nonvolatile content or Non-volatile matter content and represents the nonvolatile content in a resin solution. The addition polymerization reaction is basically synthesized in a solvent. A polymerized polymer is considered the nonvolatile content, whereas a non-polymerized monomer is considered a volatile content. As the addition polymerization reaction proceeds and the monomer is consumed, the volatile content decreases and the nonvolatile content increases. Checking an increase in the nonvolatile content to a certain value allows a reaction ratio to be estimated.

As described above, according to the present embodiment, the explanatory factors include the feature values obtained by the clustering analysis of the time-series data from the measurement instruments at the temperature rising process and the dropwise addition process. The objective factor is characterized by including either an NV or a solution viscosity. In the case where such an objective factor in the addition polymerization reaction is predicted, the prediction accuracy can be improved by including, in the explanatory factors, the feature values obtained by the clustering analysis of the time-series data from the measurement instruments at the temperature rising process and the dropwise addition process as described later. Therefore, according the present embodiment, the prediction technology related to the addition polymerization reaction can be improved.

(Configuration of Information Processing Device)

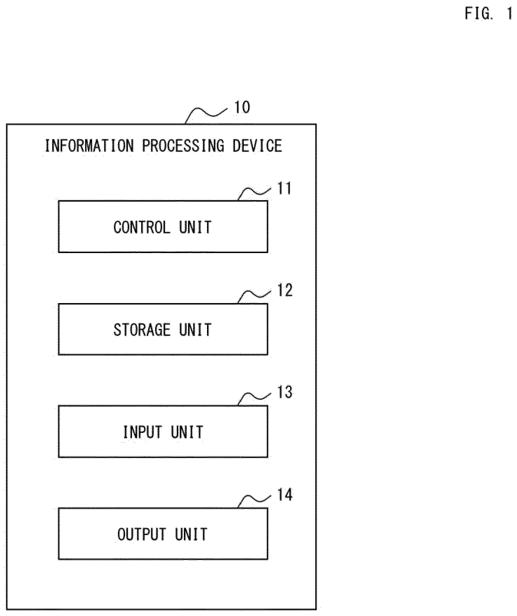

Subsequently, referring to FIG. 1, each configuration of the information processing device 10 will be described in detail. The information processing device 10 is an arbitrary device used by users. For example, personal computers, server computers, general-purpose electronic devices, or dedicated electronic devices can be employed as the information processing device 10.

As illustrated in FIG. 1, the information processing device 10 includes a control unit 11, a storage unit 12, an input unit 13, and an output unit 14.

The control unit 11 includes at least one processor, at least one dedicated circuit, or a combination thereof. The processor is a general-purpose processor such as a central processing unit (CPU) or a graphics processing unit (GPU), or a dedicated processor specialized for specific processing. The dedicated circuit is, for example, a field-programmable gate array (FPGA) or an application specific integrated circuit (ASIC). The control unit 11 executes processes associated with the operation of the information processing device 10 while controlling each part of the information processing device 10.

The storage unit 12 includes at least one semiconductor memory, at least one magnetic memory, at least one optical memory, or a combination of at least two of these memories. The semiconductor memory is, for example, a random-access memory (RAM) or a read-only memory (ROM). The RAM is, for example, a static random-access memory (SRAM) or a dynamic random-access memory (DRAM). The ROM is, for example, an electrically erasable programmable read-only memory (EEPROM). The storage unit 12 functions, for example, as a main memory device, an auxiliary memory device, or a cache memory. In the storage unit 12, data used in the operation of the information processing device 10 and data obtained by the operation of the information processing device 10 are stored.

The input unit 13 includes at least one interface for input. The interface for input is, for example, physical keys, capacitive keys, pointing devices, or touch screens integrated with displays. The interface for input may be, for example, a microphone that accepts voice input or a camera that accepts gesture input. The input unit 13 accepts operations to input data used in the operation of the information processing device 10. The input unit 13 may be connected to the information processing device 10 as an external input device instead of being provided in the information processing device 10. For example, any method such as universal serial bus (USB), high-definition multimedia interface (HDMI) (registered trademark), or Bluetooth (registered trademark) can be used as the connection method.

The output unit 14 includes at least one interface for output. The interface for output is, for example, a display that outputs information in the form of images. The display is, for example, a liquid crystal display (LCD) or an organic electroluminescence (EL) display. The output unit 14 displays and outputs data obtained by the operation of the information processing device 10. The output unit 14 may be connected to the information processing device 10 as an external output device instead of being provided in the information processing device 10. For example, any method such as USB, HDMI (registered trademark), or Bluetooth (registered trademark) can be used as the connection method.

The functions of the information processing device 10 are achieved by executing a program according to the present embodiment on a processor corresponding to the information processing device 10. In other words, the functions of the information processing device 10 are achieved by software. The program causes the computer to function as the information processing device 10 by causing the computer to execute the operations of the information processing device 10. In other words, the computer functions as the information processing device 10 by executing the operation of the information processing device 10 in accordance with the program.

In the present embodiment, the program can be recorded on a computer-readable recording medium. The computer-readable recording media include non-transient computer-readable media, for example, magnetic recording devices, optical discs, magneto-optical recording media, or semiconductor memories. Program distribution is performed by, for example, selling, assigning, or lending removal recording media such as a digital versatile disc (DVD) or a compact disc read only memory (CD-ROM) on which the program is recorded. Program distribution may also be done by storing the program in a storage on an external server and transmitting the program from the external server to other computers. The program may also be provided as a program product.

Some or all of the functions of the information processing device 10 may be achieved by a dedicated circuit corresponding to the control unit 11. In other words, some or all of the functions of the information processing device 10 may be achieved by hardware.

In the present embodiment, the storage unit 12 stores therein, for example, actual data and prediction models. The actual data and the prediction model may be stored in an external device separate from the information processing device 10. In this case, the information processing device 10 may be equipped with an interface for external communication. The interface for communication may be either an interface of a wired communication or an interface of wireless communication. In the case of the wired communication, the interface for communication is, for example, a LAN interface or USB. In the case of the wireless communication, the interface for communication is, for example, an interface compliant with mobile communication standards such as LTE, 4G, or 5G, or an interface compliant with short-range wireless communication such as Bluetooth (registered trademark). The interface for communication can receive data used in the operation of the information processing device 10 and can transmit data obtained by the operation of the information processing device 10.

(Operation of Information Processing Device)

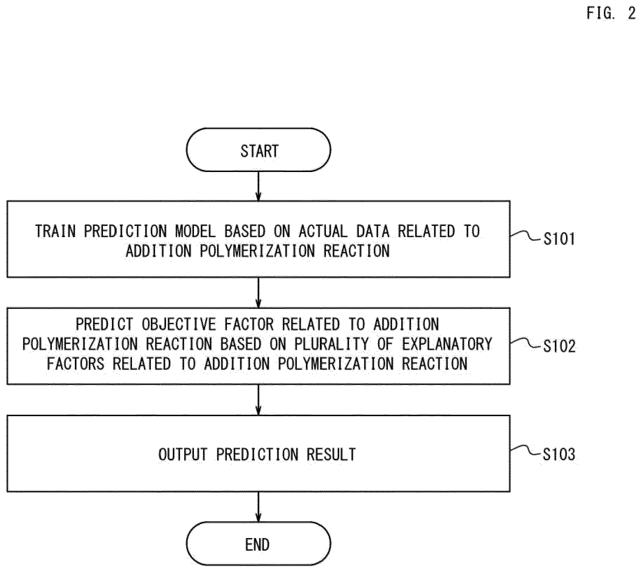

Subsequently, with reference to FIG. 2, the operation of the information processing device 10 according to the present embodiment will be described.

Step S101: The control unit 11 of the information processing device 10 trains a prediction model based on actual data related to the addition polymerization reaction. The actual data include the explanatory factors and the objective factor related to the addition polymerization reaction. The explanatory factors include the feature values obtained by the clustering analysis of the time-series data from the measurement instruments at the temperature rising process and the dropwise addition process. The objective factor includes at least either an NV or a solution viscosity. In other words, the control unit 11 trains the prediction model using these explanatory factors and objective factor included in the actual data as learning data.

Any method can be employed to acquire the actual data. For example, the control unit 11 acquires the actual data from the storage unit 12. The control unit 11 may also acquire the actual data by accepting input of the actual data from the user by the input unit 13. Alternatively, the control unit 11 may acquire such actual data from an external device that stores therein the actual data through an interface for communication.

The prediction model trained based on the learning data is cross-validated. As a result of such cross-validation, in the case where an accuracy is within a practical range, the prediction related to the addition polymerization reaction is performed using the prediction model.

Step S102: The control unit 11 predicts the objective factor related to the addition polymerization reaction based on the explanatory factors related to the addition polymerization reaction. For example, the control unit 11 may acquire the explanatory factors by accepting input of the explanatory factors from the user by the input unit 13.

Step S103: The control unit 11 outputs the objective factor predicted at Step S102 as the prediction result from the output unit 14.

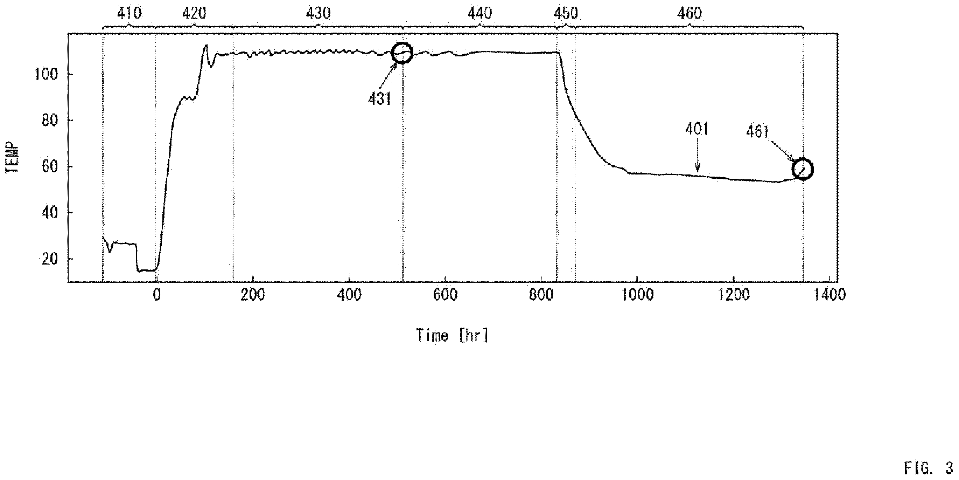

Here, in the present embodiment, the explanatory factors are characterized by including the feature values obtained by the clustering analysis of the time-series data from the measurement instruments at the temperature rising process and the dropwise addition process. FIG. 3 is a conceptual view illustrating the addition polymerization reaction process. As illustrated in FIG. 3, the addition polymerization reaction includes a charging process 410, a temperature rising process 420, a dropwise addition process 430, a holding process 440, a cooling process 450, and a post-cooling process 460. A graph 401 illustrates the temperature transition of a material to be synthesized. At the temperature rising process 420, the temperature of the material to be synthesized rises. In the holding process 440, the temperature of the material to be synthesized is kept constant. At a final stage 461 of the post-cooling process 460, the quality values of the material are analyzed.

The above analytical values in the addition polymerization reaction correspond to the objective factor in the present embodiment. Such analytical values also depend on the temperature rising process and the dropwise addition process. On the other hand, a wide variety of time-series data are involved at the temperature rising process and the dropwise addition process, and thus using all of these time-series data as the explanatory variables is not realistic. Here, the time-series data include measurement values at intervals of 1 second to 1 minute. Therefore, the present embodiment is characterized by using the feature values obtained by the clustering analysis of the time-series data from the measurement instruments at the temperature rising process and the dropwise addition process as the explanatory factors. Specifically, the clustering analysis is performed at the end of the dropwise addition process 430 in FIG. 3 and, the reaction prediction is performed all at once for approximately 5 hours (the time of the holding process 440) ahead to determine the cooling timing in the cooling process 450 from the timing (at the end point of the dropwise addition process 431) when all the explanatory factors are collected.

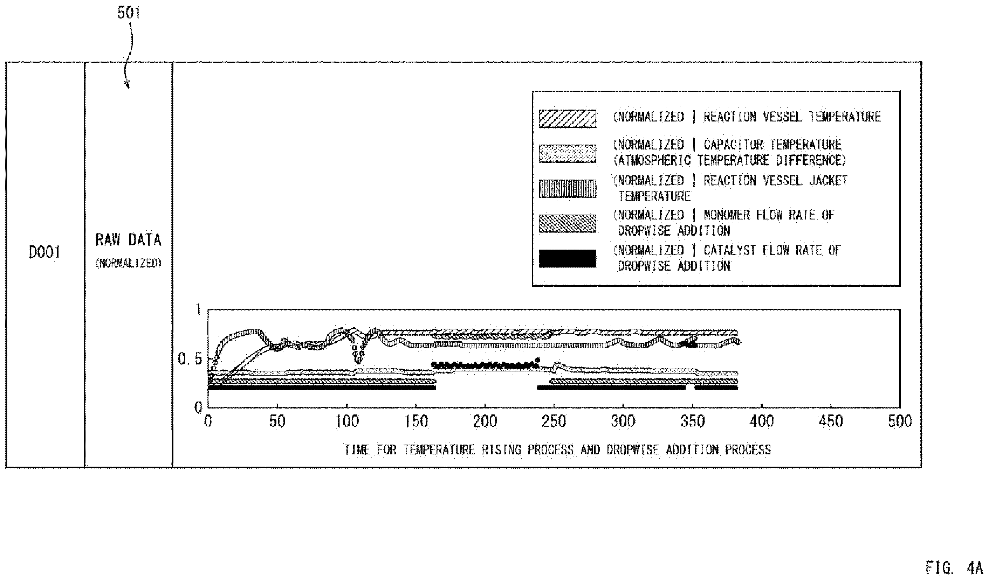

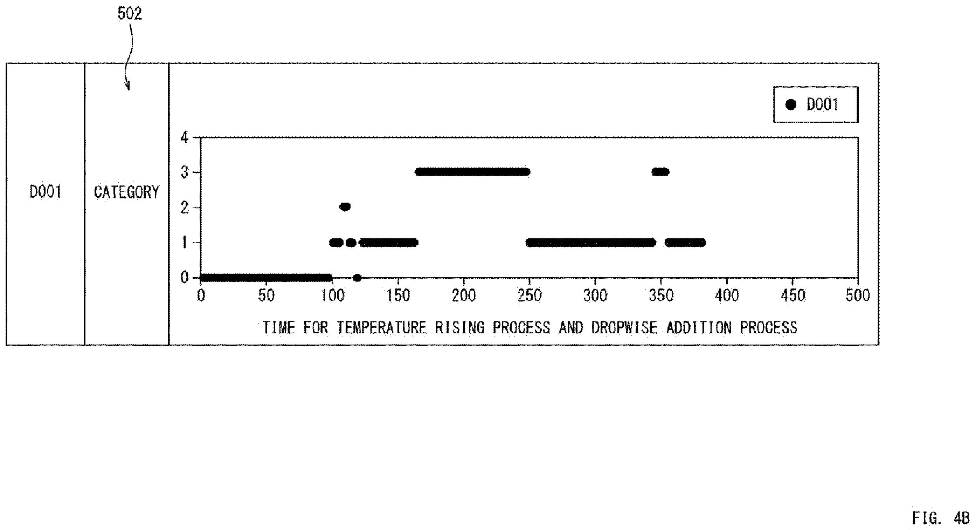

FIG. 4A and FIG. 4B illustrate a method for calculating the feature values in a certain lot (a lot having a lot number of D001). An item 501 in FIG. 4A represents the time transition of each piece of the data pertaining to the temperature rising process and the dropwise addition process related to such a lot. Such data include data related to a reaction vessel temperature, a capacitor temperature (atmospheric temperature difference), a reaction vessel jacket temperature, a monomer flow rate of dropwise addition, and a catalyst flow rate of dropwise addition. Each piece of the data in the item 501 is normalized. An item 502 in FIG. 4B represents the time transition of the categories related to the temperature rising process and the dropwise addition process. In the present embodiment, the categories include five steps of 0-4. Specifically, the categories are determined by the clustering analysis of the time-series data from the measurement instruments at the temperature rising process and the dropwise addition process. Based on these categories, the feature values at the temperature rising process and the dropwise addition process are determined. According to the present invention, the feature values are determined based on the time rate of the categories. The time rate of the categories refers to a value obtained by dividing the cumulative residence time of each category by the overall time. As described above, in the present embodiment, the feature values are provided by the clustering analysis at the temperature rising process and the dropwise addition process.

As described above, according to the present embodiment, the explanatory factors include the feature values obtained by the clustering analysis of the time-series data from the measurement instruments at the temperature rising process and the dropwise addition process. The objective factor is characterized by including either an NV or a solution viscosity. In the case where the prediction related to the addition polymerization reaction is performed, the accuracy of the prediction model can be improved by including, in the explanatory factors, the feature values obtained by the clustering analysis of the temperature rising process and the dropwise addition process. Therefore, according the present embodiment, the prediction technology related to the addition polymerization reaction can be improved.

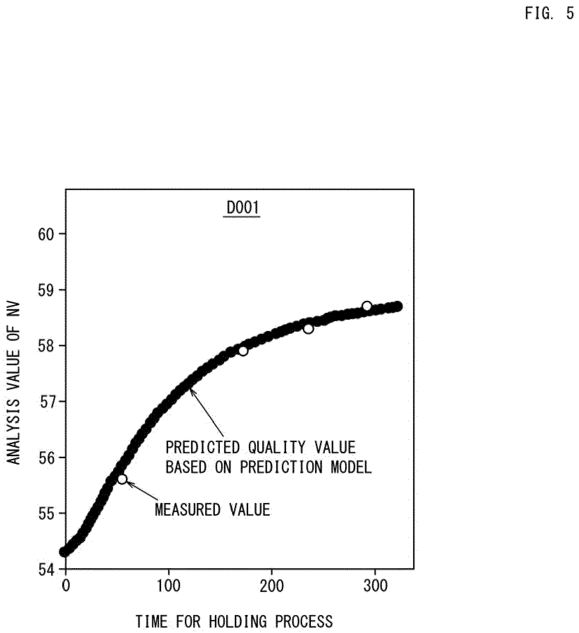

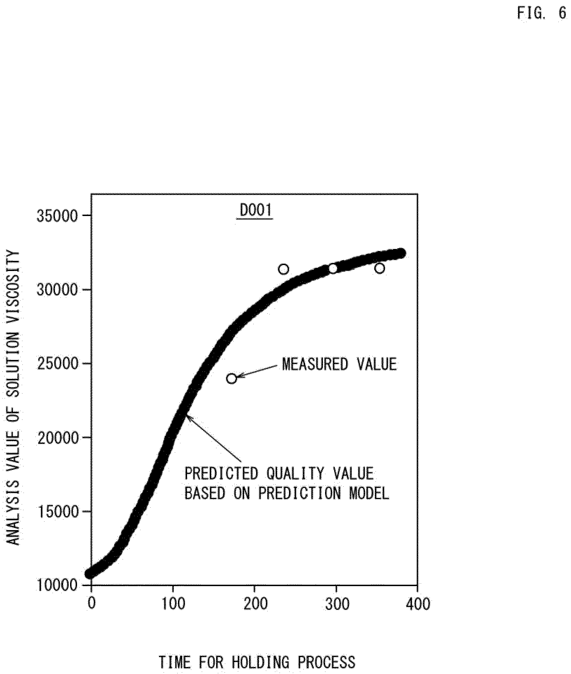

FIG. 5 and FIG. 6 illustrate an example of the accuracy verification results of the prediction model according to the present embodiment. FIG. 5 is a graph illustrating the NV and the measured values of a certain lot (lot number D001) of an acrylic predicted by the prediction model. As illustrated in FIG. 5, the NV predicted by the prediction model approximately agrees with the measured values. FIG. 6 is a graph illustrating the solution viscosity and the measured values of the above lot of the acrylic predicted by the prediction model. As illustrated in FIG. 6, the solution viscosity predicted by the prediction model approximately agrees with the measured values. Therefore, it is found that the accuracy of the prediction model is sufficiently high.

Here, the explanatory factors may include theoretical values in a reaction physics model (reaction physics model theoretical values). As described above, the reaction physics model may serve as explanatory factors as a baseline of the reaction characteristics. Similarly, the explanatory factors may include the amount of a charged raw material monomer, the amount of a charged raw material solvent, the amount of a charged initiator, the reaction vessel temperature, the capacitor temperature, the reaction vessel jacket temperature, and the like.

Here, the prediction model according to the present embodiment may be, for example, a neural network model. In the case where the prediction model is the neural network model, the coefficients of activation functions of the neural network model may be different between an intermediate layer and an output layer. For example, the coefficient of the activation function related to the intermediate layer is larger than the coefficient of the activation function related to the output layer.

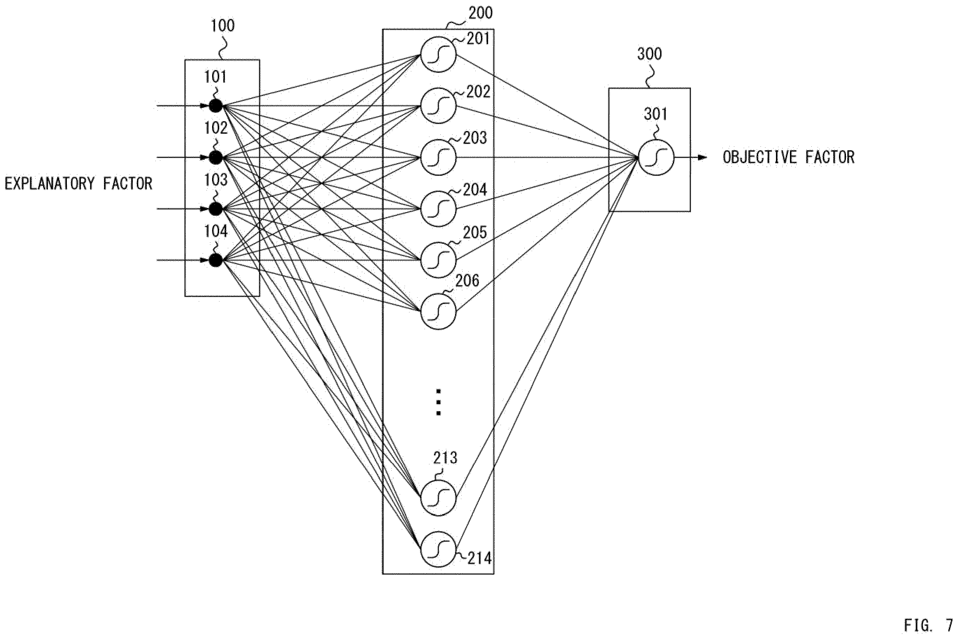

FIG. 7 is a conceptual diagram of the neural network model according to the present embodiment. Such a neural network model includes an input layer 100, an intermediate layer 200, and an output layer 300. The neural network model in the present embodiment is fully connected. In the present embodiment, the number of layers in the neural network model is, for example, 2. Such number of layers is the number of layers excluding the input layer. By setting the number of layers in the neural network model to 2, a model configuration can be prevented from becoming inappropriate to the physical phenomena in the addition polymerization reaction. In other words, the number of layers of neural network model can be kept to minimum necessary, whereby the model configuration suitable for the physical phenomena in the addition polymerization reaction can be achieved. The number of layers of the neural network model according to the present embodiment is not limited to this number of layers, and may be three layers or more. In the case where the number of layers in the neural network model is three or more, as the layer of the neural network model becomes more front side, the coefficient of the activation function may be set larger.

The input layer 100 includes a plurality of elements 101 to 104 (also referred to as input elements 101 to 104). In the neural network model illustrated in FIG. 7, the number of the input elements is 4. The input elements 101 to 104 are also referred to as the first to fourth elements, respectively. In the input elements 101 to 104, each of the explanatory factors is input. The number of input elements is not limited to this and may be less than 4 or 5 or more.

The intermediate layer 200 includes a plurality of elements 201 to 214 (also referred to as intermediate elements 201 to 214). In the neural network model illustrated in FIG. 7, the number of the intermediate elements is 14. The intermediate elements 201 to 214 are also referred to as the first to fourteenth elements, respectively. The number of intermediate elements is not limited to this, and may be less than 14 or 15 or more.

The output layer 300 includes an element 301 (an output element 301). In the neural network model illustrated in FIG. 7, the number of the output elements is 1. The output element 301 is also referred to as the first element The number of the output elements is not limited to this, and may be 2 or more.

The values input from the input elements 101 to 104 of the input layer 100 to the intermediate elements 201 to 214 of the intermediate layer 200 are converted in the intermediate layer 200 based on the activation function related to the intermediate layer 200. The converted values are output to the element 301 of the output layer 300. The activation function related to the intermediate layer 200 is, for example, a sigmoid function. The values input from the intermediate elements 201 to 214 of the intermediate layer 200 to the output element 301 of the output layer 300 are converted in the output layer 300 based on the activation function related to the output layer 300 and output. The activation function related to the output layer 300 is, for example, the sigmoid function. Specifically, the activation functions related to the intermediate layer and the output layer are, for example, the respective sigmoid functions determined by the following formulas (1) and (2).

[Mathematical Formula 1]

$$f^1\left(u_j^1\right) = \frac{1}{1+e^{-a_1 u_j^1}} \quad (1)$$

$$f^2\left(u_j^2\right) = \frac{1}{1+e^{-a_2 u_j^2}} \quad (2)$$

Here, $f^1\left(u_j^1\right)$ is the activation function related to the intermediate layer 200, $a_1$ is the coefficient of the activation function related to the intermediate layer 200, and $u_j^1$ is the input value input to the j-th element of the intermediate layer 200. In the example in FIG. 7, the number of the intermediate elements is 14, and this j takes the value from 1 to 14. $f^2\left(u_j^2\right)$ is the activation function related to the output layer 300, $a_2$ is the coefficient of the activation function related to the output layer 300, and $u_j^2$ is the input value input to the j-th element of the output layer 300. In the example in FIG. 7, j is 1 because the number of the output elements is 1. As described above, in the neural network model according to the present embodiment, the coefficient of the activation function related to the intermediate layer 200 is larger than the coefficient of the activation function related to the output layer 300. In other words, $a_1$ and $a_2$ in the neural network model according to the present embodiment satisfy $a_1 > a_2$.

In the neural network model according to the present embodiment, the coefficient of the activation function related to the intermediate layer is larger than the coefficient of the activation function related to the output layer. This allows the configuration of the neural network model to be optimized at the time of performing the prediction related to the addition polymerization reaction. Specifically, in the neural network model for performing the prediction related to the addition polymerization reaction, change in the explanatory factors is desirably viewed as obvious change. Therefore, by setting the coefficient of the activation functions related to the intermediate layer larger than the coefficient of the activation functions related to the output layer, the change in the input values to the intermediate layer can be transmitted to the output layer as the obvious change. On the other hand, in the output layer of the neural network model for performing the prediction related to the addition polymerization reaction, the values of the training data and the objective factor are required to be converged. Therefore, the coefficient of the activation function related to the output layer is set smaller than the coefficient of the activation function related to the intermediate layer. By doing so, the value of the objective factor output from the output layer is finely adjusted.

By setting the coefficients of the activation functions between the intermediate layer and the output layer to be different, the learning process of the neural network model is optimized. Specifically, the updated amounts of the weight variables in the output layer and the intermediate layer during the learning process can be adjusted by changing the coefficient of the activation function. In addition, updating the weight variables provides a significant impact on the learning process. Therefore, the learning process may be optimized based on the adjustment of the updated amounts.

Specifically, in the neural network model at the time of performing prediction related to the addition polymerization reaction, the updated amount of the weight variables related to the intermediate layer is preferably set to be relatively large. This allows the weight variables in the intermediate layer to vary more significantly during the learning process, and thus changes in the input values to the intermediate layer to be transferred to the output layer as obvious changes. On the other hand, the updated amount of weight variables related to the output layer is preferably set to be relatively small. This allows the weight variables in the output layer to vary less during the learning process and thus the values of the training data and the objective factor to be easily converged. In addition, by satisfying $a^l > a^L$, an arbitrary smooth function can be approximated with sufficient accuracy, eliminating the need to inadvertently increase the number of intermediate layers. This allows sufficient accuracy to be obtained even when the intermediate layer is one layer. Preparing fewer intermediate layers directly leads to reduction in generation of over-fitting and thus provides a secondary effect on stability of the learning process and, in addition, robustness of the model.

The case where the activation functions of the intermediate layer and the output layer are sigmoid functions is described in the present embodiment. However, the activation functions are not limited to the sigmoid functions. For example, the activation functions of the intermediate layer and the output layer may be functions such as a hyperbolic tangent function (tanh function) and a ramp functions (ReLU).

Reference Signs List

| 10 | information processing device |
|---|---|
| 11 | control unit |
| 12 | storage unit |
| 13 | input unit |
| 14 | output unit |
| 100 | input layer |
| 200 | intermediate layer |
| 300 | output layer |
| 101 to 104, 201 to 214, and 301 | element |
| 401 | graph |
| 410 | charging process |
| 420 | temperature rising process |
| 430 | dropwise addition process |
| 431 | end point of temperature rising process |
| 440 | holding process |
| 450 | cooling process |
| 460 | post-cooling process |
| 461 | final stage |
| 501 and 502 | item |

**Claims**

1. A method for performing prediction related to an addition polymerization reaction executed by an information processing device (10), the method comprising:

   a step of training (S101) a prediction model based on actual data comprising a plurality of explanatory factors and an objective factor related to the addition polymerization reaction; and
   a step of predicting (S102) the objective factor during the addition polymerization reaction based on the explanatory factors related to the addition polymerization reaction by the prediction model, wherein
   the addition polymerization reaction includes a charging process (410), a temperature rising process (420), a dropwise addition process (430), a holding process (440), a cooling process (450), and a post-cooling process (460),
   the explanatory factors include a plurality of feature values obtained by clustering analysis of normalized time-series data obtained by normalizing time-series data from a plurality of measurement instruments at the temperature rising process (420) and the dropwise addition process (430),
   the time-series data from the plurality of measurement instruments at the temperature rising process and the dropwise addition process comprises a plurality of data types out of the following types: a reaction vessel temperature, a capacitor temperature, an atmospheric temperature difference, a reaction vessel jacket temperature, a monomer flow rate of dropwise addition, and a catalyst flow rate of dropwise addition,
   the clustering analysis comprises clustering the normalized time-series data comprising the plurality of data types in categories, and the feature values in the temperature rising process and the dropwise addition process are determined based on a time rate of the categories,
   the step of predicting comprises predicting the objective factor during the holding process (440), and
   the objective factor includes at least either a non-volatile content, NV, representing the nonvolatile content in a resin solution, or a solution viscosity.

2. The method according to claim 1, wherein the explanatory factors include theoretical values in a reaction physics model.

3. The method according to claim 1, wherein the addition polymerization reaction is an addition polymerization reaction of an acrylic.

4. The method according to claim 1, wherein the prediction model is a neural network model including an input layer (100), an intermediate layer (200), and an output layer (300) and a coefficient of an activation function related to the intermediate layer is larger than a coefficient of an activation function related to the output layer.

5. An information processing device (10) performing prediction related to an addition polymerization reaction, the information processing device comprising a control unit (11), wherein

the control unit (11)

trains a prediction model based on actual data comprising a plurality of explanatory factors and an objective factor related to the addition polymerization reaction, and

predicts the objective factor during the addition polymerization reaction based on the explanatory factors related to the addition polymerization reaction by the prediction model, and

the addition polymerization reaction includes a charging process (410), a temperature rising process (420), a dropwise addition process (430), a holding process (440), a cooling process (450), and a post-cooling process (460),

the explanatory factors include a plurality of feature values obtained by clustering analysis of normalized time-series data obtained by normalizing time-series data from a plurality of measurement instruments at the temperature rising process (420) and the dropwise addition process (430),

the time-series data from the plurality of measurement instruments at the temperature rising process and the dropwise addition process comprises a plurality of data types out of the following types: a reaction vessel temperature, a capacitor temperature, an atmospheric temperature difference, a reaction vessel jacket temperature, a monomer flow rate of dropwise addition, and a catalyst flow rate of dropwise addition,

the clustering analysis comprises clustering the normalized time-series data comprising the plurality of data types in categories, and the feature values in the temperature rising process and the dropwise addition process are determined based on a time rate of the categories,

the control unit predicts the objective factor during the holding process (440), and

the objective factor includes at least either a non-volatile content, NV, representing the nonvolatile content in a resin solution, or a solution viscosity.

6. A non-transitory computer-readable recording medium storing therein instructions, in which when the instructions are executed by a processor of an information processing device performing prediction related to an addition polymerization reaction, the instructions cause the processor to execute:

training of a prediction model based on actual data comprising a plurality of explanatory factors and an objective factor related to the addition polymerization reaction; and

prediction of the objective factor during the addition polymerization reaction based on the explanatory factors related to the addition polymerization reaction by the prediction model, wherein

the addition polymerization reaction includes a charging process (410), a temperature rising process (420), a dropwise addition process (430), a holding process (440), a cooling process (450), and a post-cooling process (460),

the explanatory factors include a plurality of feature values obtained by clustering analysis of normalized time-series data obtained by normalizing time-series data from a plurality of measurement instruments at the temperature rising process (420) and the dropwise addition process (430),

the time-series data from the plurality of measurement instruments at the temperature rising process and the dropwise addition process comprises a plurality of data types out of the following types: a reaction vessel temperature, a capacitor temperature, an atmospheric temperature difference, a reaction vessel jacket temperature, a monomer flow rate of dropwise addition, and a catalyst flow rate of dropwise addition,

the clustering analysis comprises clustering the normalized time-series data comprising the plurality of data types in categories, and the feature values in the temperature rising process and the dropwise addition process are determined based on a time rate of the categories,

the prediction comprises prediction of the objective factor during the holding process (440), and

the objective factor includes at least either a non-volatile content, NV, representing the nonvolatile content in a resin solution, or a solution viscosity.

**Patentansprüche**

1. Verfahren zum Durchführen einer Prädiktion hinsichtlich einer Additionspolymerisationsreaktion, die durch eine Informationsverarbeitungseinrichtung (10) ausgeführt wird, wobei das Verfahren umfasst:

   einen Schritt eines Trainierens (S101) eines Prädiktionsmodells beruhend auf tatsächlichen Daten, die eine Vielzahl von Erläuterungsfaktoren und einen Zielfaktor hinsichtlich der Additionspolymerisationsreaktion umfassen; und

   einen Schritt eines Prognostizierens (S102) des Zielfaktors während der Additionspolymerisationsreaktion beruhend auf den erläuternden Faktoren hinsichtlich der Additionspolymerisationsreaktion durch das Prädiktionsmodell, wobei

   die Additionspolymerisationsreaktion einen Beschickungsprozess (410), einen Temperaturanstiegsprozess (420), einen Eintropfprozess (430), einen Halteprozess (440), einen Kühlprozess (450) und einen Post-Kühlprozess (460) enthält,

   die erläuternden Faktoren eine Vielzahl von Merkmalswerten enthalten, die durch eine Clusteranalyse von normalisierten Zeitreihendaten erhalten werden, die durch Normalisieren von Zeitreihendaten von einer Vielzahl von Messinstrumenten bei dem Temperaturanstiegsprozess (420) und dem Eintropfprozess (430) erhalten werden,

   die Zeitreihendaten von der Vielzahl von Messinstrumenten bei dem Temperaturanstiegsprozess und dem Eintropfprozess eine Vielzahl von Datentypen aus den folgenden Typen umfassen: Reaktionsbehältertemperatur, Kondensatortemperatur, Lufttemperaturdifferenz, Reaktionsbehältermanteltemperatur, Monomerdurchfluss des Eintropfens und Katalysatordurchfluss des Eintropfens,

   die Clusteranalyse ein Clustern der normalisierten Zeitreihendaten, die die Vielzahl von Datentypen umfassen, in Kategorien umfasst, und die Merkmalswerte bei dem Temperaturanstiegsprozess und dem Eintropfprozess beruhend auf einer Zeitrate der Kategorien bestimmt werden,

   der Schritt des Prognostizierens ein Prognostizieren des Zielfaktors während des Halteprozesses (440) umfasst, und

   der Zielfaktor zumindest entweder einen nichtflüchtigen Gehalt, NV, der den nichtflüchtigen Gehalt in einer Harzlösung darstellt, oder eine Lösungsviskosität enthält.

2. Verfahren nach Anspruch 1, wobei die erläuternden Faktoren theoretische Werte in einem physikalischen Reaktionsmodell enthalten.

3. Verfahren nach Anspruch 1, wobei die Additionspolymerisationsreaktion eine Additionspolymerisationsreaktion von Acryl ist.

4. Verfahren nach Anspruch 1, wobei das Prädiktionsmodell ein neuronales Netzwerkmodell ist, das eine Eingabeschicht (100), eine Zwischenschicht (200) und eine Ausgabeschicht (300) enthält, und ein Koeffizient einer Aktivierungsfunktion hinsichtlich der Zwischenschicht größer als ein Koeffizient einer Aktivierungsfunktion hinsichtlich der Ausgabeschicht ist.

5. Informationsverarbeitungseinrichtung (10), die eine Prädiktion hinsichtlich einer Additionspolymerisationsreaktion durchführt, wobei die Informationsverarbeitungseinrichtung eine Steuereinheit (11) umfasst, wobei

   die Steuereinheit (11)

   ein Prädiktionsmodell beruhend auf tatsächlichen Daten trainiert, die eine Vielzahl von erläuternden Faktoren und einen Zielfaktor hinsichtlich der Additionspolymerisationsreaktion umfassen, und

   den Zielfaktor während der Additionspolymerisationsreaktion beruhend auf den erläuternden Faktoren hinsichtlich der Additionspolymerisationsreaktion durch das Prädiktionsmodell prognostiziert, und

   die Additionspolymerisationsreaktion einen Beschickungsprozess (410), einen Temperaturanstiegsprozess (420), einen Eintropfprozess (430), einen Halteprozess (440), einen Kühlprozess (450) und einen Post-Kühlprozess (460) enthält,

   die erläuternden Faktoren eine Vielzahl von Merkmalswerten enthalten, die durch eine Clusteranalyse von normalisierten Zeitreihendaten erhalten werden, die durch Normalisieren von Zeitreihendaten von einer Vielzahl von Messinstrumenten bei dem Temperaturanstiegsprozess (420) und dem Eintropfprozess (430) erhalten werden,

die Zeitreihendaten von der Vielzahl von Messinstrumenten bei dem Temperaturanstiegsprozess und dem Eintropfprozess eine Vielzahl von Datentypen aus den folgenden Typen umfassen: Reaktionsbehältertemperatur, Kondensatortemperatur, Lufttemperaturdifferenz, Reaktionsbehältermanteltemperatur, Monomerdurchfluss des Eintropfens und Katalysatordurchfluss des Eintropfens,

die Clusteranalyse ein Clustern der normalisierten Zeitreihendaten, die die Vielzahl von Datentypen umfassen, in Kategorien umfasst, und die Merkmalswerte bei dem Temperaturanstiegsprozess und dem Eintropfprozess beruhend auf einer Zeitrate der Kategorien bestimmt werden,

die Steuereinheit den Zielfaktor während des Halteprozesses (440) prognostiziert, und

der Zielfaktor zumindest entweder einen nichtflüchtigen Gehalt, NV, der den nichtflüchtigen Gehalt in einer Harzlösung darstellt, oder eine Lösungsviskosität enthält.

6. Nichtflüchtiges computerlesbares Aufzeichnungsmedium, das Instruktionen speichert, wobei, wenn die Instruktionen durch einen Prozessor einer Informationsverarbeitungseinrichtung ausgeführt werden, die eine Prädiktion hinsichtlich einer Additionspolymerisationsreaktion durchführt, die Instruktionen den Prozessor veranlassen, Folgendes auszuführen:

Trainieren eines Prädiktionsmodells beruhend auf tatsächlichen Daten, die eine Vielzahl von erläuternden Faktoren und einen Zielfaktor hinsichtlich der Additionspolymerisationsreaktion umfassen; und

eine Prädiktion des Zielfaktors während der Additionspolymerisationsreaktion beruhend auf den erläuternden Faktoren hinsichtlich der Additionspolymerisationsreaktion durch das Prädiktionsmodell, wobei

die Additionspolymerisationsreaktion einen Beschickungsprozess (410), einen Temperaturanstiegsprozess (420), einen Eintropfprozess (430), einen Halteprozess (440), einen Kühlprozess (450) und einen Post-Kühlprozess (460) enthält,

die erläuternden Faktoren eine Vielzahl von Merkmalswerten enthalten, die durch eine Clusteranalyse von normalisierten Zeitreihendaten erhalten werden, die durch Normalisieren von Zeitreihendaten von einer Vielzahl von Messinstrumenten bei dem Temperaturanstiegsprozess (420) und dem Eintropfprozess (430) erhalten werden,

die Zeitreihendaten von der Vielzahl von Messinstrumenten bei dem Temperaturanstiegsprozess und dem Eintropfprozess eine Vielzahl von Datentypen aus den folgenden Typen umfassen: Reaktionsbehältertemperatur, Kondensatortemperatur, Lufttemperaturdifferenz, Reaktionsbehältermanteltemperatur, Monomerdurchfluss des Eintropfens und Katalysatordurchfluss des Eintropfens,

die Clusteranalyse ein Clustern der normalisierten Zeitreihendaten, die die Vielzahl von Datentypen umfassen, in Kategorien umfasst, und die Merkmalswerte bei dem Temperaturanstiegsprozess und dem Eintropfprozess beruhend auf einer Zeitrate der Kategorien bestimmt werden,

die Prädiktion eine Prädiktion des Zielfaktors während des Halteprozesses (440) umfasst, und

der Zielfaktor zumindest entweder einen nichtflüchtigen Gehalt, NV, der den nichtflüchtigen Gehalt in einer Harzlösung darstellt, oder eine Lösungsviskosität enthält.

## Revendications

1. Procédé destiné à effectuer une prédiction relative à une réaction de polymérisation par addition exécuté par un dispositif de traitement d'informations (10), le procédé comprenant :

une étape d'entraînement (S101) d'un modèle de prédiction sur la base de données réelles comprenant une pluralité de facteurs explicatifs et un facteur objectif relatifs à la réaction de polymérisation par addition ; et

une étape de prédiction (S102) du facteur objectif pendant la réaction de polymérisation par addition sur la base des facteurs explicatifs relatifs à la réaction de polymérisation par addition par le modèle de prédiction, dans lequel

la réaction de polymérisation par addition comporte un processus de chargement (410), un processus de montée en température (420), un processus d'ajout goutte à goutte (430), un processus de maintien (440), un processus de refroidissement (450), et un processus de post-refroidissement (460),

les facteurs explicatifs comportent une pluralité de valeurs caractéristiques obtenues par analyse par regroupement de données de séries temporelles normalisées obtenues par normalisation de données de séries temporelles provenant d'une pluralité d'instruments de mesure lors du processus de montée en température (420) et du processus d'ajout goutte à goutte (430),

les données de séries temporelles provenant de la pluralité d'instruments de mesure lors du processus de montée en température et du processus d'ajout goutte à goutte comprennent une pluralité de types de données parmi les types suivants : une température de cuve de réaction, une température de condensateur, une différence de température atmosphérique, une température de chemise de cuve de réaction, un débit de monomère d'ajout goutte à goutte, et un débit de catalyseur d'ajout goutte à goutte,

l'analyse par regroupement comprend le regroupement des données de séries temporelles normalisées comprenant la pluralité de types de données en catégories, et les valeurs caractéristiques dans le processus de montée en température et le processus d'ajout goutte à goutte sont déterminées sur la base d'une proportion temporelle des catégories,

l'étape de prédiction comprend la prédiction du facteur objectif pendant le processus de maintien (440), et

le facteur objectif comporte au moins soit une teneur en composés non volatils, NV, représentant la teneur en composés non volatils dans une solution de résine, soit une viscosité en solution.

2. Procédé selon la revendication 1, dans lequel les facteurs explicatifs comportent des valeurs théoriques dans un modèle de physique de réaction.

3. Procédé selon la revendication 1, dans lequel la réaction de polymérisation par addition est une réaction de polymérisation par addition d'un acrylique.

4. Procédé selon la revendication 1, dans lequel le modèle de prédiction est un modèle de réseau neuronal comportant une couche d'entrée (100), une couche intermédiaire (200) et une couche de sortie (300), et un coefficient d'une fonction d'activation relative à la couche intermédiaire est supérieur à un coefficient d'une fonction d'activation relative à la couche de sortie.

5. Dispositif de traitement d'informations (10) effectuant une prédiction relative à une réaction de polymérisation par addition, le dispositif de traitement d'informations comprenant une unité de commande (11),

dans lequel l'unité de commande (11)
entraîne un modèle de prédiction sur la base de données réelles comprenant une pluralité de facteurs explicatifs et un facteur objectif relatifs à la réaction de polymérisation par addition, et prédit le facteur objectif pendant la réaction de polymérisation par addition sur la base des facteurs explicatifs relatifs à la réaction de polymérisation par addition par le modèle de prédiction, et

la réaction de polymérisation par addition comporte un processus de chargement (410), un processus de montée en température (420), un processus d'ajout goutte à goutte (430), un processus de maintien (440), un processus de refroidissement (450), et un processus de post-refroidissement (460),

les facteurs explicatifs comportent une pluralité de valeurs caractéristiques obtenues par analyse par regroupement de données de séries temporelles normalisées obtenues par normalisation de données de séries temporelles provenant d'une pluralité d'instruments de mesure lors du processus de montée en température (420) et du processus d'ajout goutte à goutte (430),

les données de séries temporelles provenant de la pluralité d'instruments de mesure lors du processus de montée en température et du processus d'ajout goutte à goutte comprennent une pluralité de types de données parmi les types suivants : une température de cuve de réaction, une température de condensateur, une différence de température atmosphérique, une température de chemise de cuve de réaction, un débit de monomère d'ajout goutte à goutte, et un débit de catalyseur d'ajout goutte à goutte,

l'analyse par regroupement comprend le regroupement des données de séries temporelles normalisées comprenant la pluralité de types de données en catégories, et les valeurs caractéristiques dans le processus de montée en température et le processus d'ajout goutte à goutte sont déterminées sur la base d'une proportion temporelle des catégories,

l'unité de commande prédit le facteur objectif pendant le processus de maintien (440), et

le facteur objectif comporte au moins soit une teneur en composés non volatils, NV, représentant la teneur en composés non volatils dans une solution de résine, soit une viscosité en solution.

6. Support d'enregistrement non transitoire lisible par ordinateur stockant des instructions, dans lequel, lorsque les instructions sont exécutées par un processeur d'un dispositif de traitement d'informations effectuant une prédiction relative à une réaction de polymérisation par addition, les instructions amènent le processeur à exécuter :

l'entraînement d'un modèle de prédiction sur la base de données réelles comprenant une pluralité de facteurs explicatifs et un facteur objectif relatifs à la réaction de polymérisation par addition ; et la prédiction du facteur objectif pendant la réaction de polymérisation par addition sur la base des facteurs explicatifs relatifs à la réaction de polymérisation par addition par le modèle de prédiction, dans lequel

la réaction de polymérisation par addition comporte un processus de chargement (410), un processus de montée en température (420), un processus d'ajout goutte à goutte (430), un processus de maintien (440), un processus de refroidissement (450), et un processus de post-refroidissement (460),

les facteurs explicatifs comportent une pluralité de valeurs caractéristiques obtenues par analyse par regroupement de données de séries temporelles normalisées obtenues par normalisation de données de séries temporelles provenant d'une pluralité d'instruments de mesure lors du processus de montée en température (420) et du processus d'ajout goutte à goutte (430),

les données de séries temporelles provenant de la pluralité d'instruments de mesure lors du processus de montée en température et du processus d'ajout goutte à goutte comprennent une pluralité de types de données parmi les types suivants : une température de cuve de réaction,

une température de condensateur, une différence de température atmosphérique, une température de chemise de cuve de réaction, un débit de monomère d'ajout goutte à goutte, et un débit de catalyseur d'ajout goutte à goutte,

l'analyse par regroupement comprend le regroupement des données de séries temporelles normalisées comprenant la pluralité de types de données en catégories, et les valeurs caractéristiques dans le processus de montée en température et le processus d'ajout goutte à goutte sont déterminées sur la base d'une proportion temporelle des catégories,

la prédiction comprend une prédiction du facteur objectif pendant le processus de maintien (440), et

le facteur objectif comporte au moins soit une teneur en composés non volatils, NV, représentant la teneur en composés non volatils dans une solution de résine, soit une viscosité en solution.

FIG. 1

```
                                              10
┌──────────────────────────────────────────┐
│      INFORMATION PROCESSING DEVICE         │
│                                     11     │
│   ┌──────────────────────────────┐         │
│   │        CONTROL UNIT          │         │
│   └──────────────────────────────┘         │
│                                     12     │
│   ┌──────────────────────────────┐         │
│   │        STORAGE UNIT          │         │
│   └──────────────────────────────┘         │
│                                     13     │
│   ┌──────────────────────────────┐         │
│   │         INPUT UNIT           │         │
│   └──────────────────────────────┘         │
│                                     14     │
│   ┌──────────────────────────────┐         │
│   │        OUTPUT UNIT           │         │
│   └──────────────────────────────┘         │
│                                            │
└──────────────────────────────────────────┘
```

FIG. 2

```
        ┌─────────────┐
        │    START    │
        └─────────────┘
               │
               ▼
┌──────────────────────────────────────────────┐
│  TRAIN PREDICTION MODEL BASED ON ACTUAL DATA  │
│  RELATED TO ADDITION POLYMERIZATION REACTION  │──S101
└──────────────────────────────────────────────┘
               │
               ▼
┌──────────────────────────────────────────────┐
│  PREDICT OBJECTIVE FACTOR RELATED TO ADDITION │
│  POLYMERIZATION REACTION BASED ON PLURALITY OF│──S102
│  EXPLANATORY FACTORS RELATED TO ADDITION      │
│  POLYMERIZATION REACTION                      │
└──────────────────────────────────────────────┘
               │
               ▼
┌──────────────────────────────────────────────┐
│          OUTPUT PREDICTION RESULT             │──S103
└──────────────────────────────────────────────┘
               │
               ▼
        ┌─────────────┐
        │     END     │
        └─────────────┘
```

FIG. 3

| D001 | RAW DATA (NORMALIZED) | |
|---|---|---|

Legend:
- (NORMALIZED | REACTION VESSEL TEMPERATURE
- (NORMALIZED | CAPACITOR TEMPERATURE (ATMOSPHERIC TEMPERATURE DIFFERENCE)
- (NORMALIZED | REACTION VESSEL JACKET TEMPERATURE
- (NORMALIZED | MONOMER FLOW RATE OF DROPWISE ADDITION
- (NORMALIZED | CATALYST FLOW RATE OF DROPWISE ADDITION

TIME FOR TEMPERATURE RISING PROCESS AND DROPWISE ADDITION PROCESS

501

FIG. 4A

502

| D001 | CATEGORY | |

EP 4 461 754 B1

FIG. 4B

FIG. 5

FIG. 6

FIG. 7

EP 4 461 754 B1

**EP 4 461 754 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023048922 A
- WO 2003026791 A
- WO 2022004880 A1

**Non-patent literature cited in the description**

- Design and Applications of Soft Sensors in Polymer Processing: A Review. **ABEYKOON CHAMIL ED**. IEEE SENSORS JOURNAL. IEEE, 15 April 2019, vol. 19, 2801-2813
- Forecasting the Intrinsic Viscosity of Polyester Based on Improved Extreme Learning Machine. **YIN ZHANGQI et al.** 2019 IEEE INTERNATIONAL CONFERENCE ON ARTIFICIAL INTELLIGENCE AND COMPUTER APPLICATIONS (ICAICA). IEEE, 29 March 2019, 241-246
- Online prediction based on the Copula function for the intrinsic viscosity of polyester fibre. **JINTAO SHI et al.** CANADIAN JOURNAL OF CHEMICAL ENGINEERING. WILEY SUBSCRIPTION SERVICES, INC, 30 May 2022, vol. 101, 1440-1454
- Fractal-based combined kernel function model for the polyester polymerization process. **GENG JUNXIAN et al.** 2021 33RD CHINESE CONTROL AND DECISION CONFERENCE (CCDC). IEEE, 22 May 2021, 656-661